# EUROPÄISCHE PATENTANMELDUNG

(11) **EP 3 932 371 A2**
(43) Veröffentlichungstag der Anmeldung: **05.01.2022**
(21) Anmeldenummer: 21178443.4
(22) Anmeldetag: 09.06.2021
(51) Int. Cl.: A61F 2/50, A61F 2/76, A61F 2/78, A61F 2/80, B33Y 10/00

(54) **GURTEINZUGSSYSTEM FÜR EINEN PROTHESENSCHAFT UND VERRIEGELUNGSTASSE, MONTAGEADAPTER, SCHIEBESTOPFEN HIERFÜR**

(30) Priorität: 30.06.2020 DE 102020003934
(71) Anmelder: OT Supply GmbH, 86529 Schrobenhausen (DE)
(72) Erfinder: DALLMAYER, Robert, 86529 Schrobenhausen (DE); KURZHALS, Christoph, 86529 Schrobenhausen (DE); SCHASER, Werner, 86529 Schrobenhausen (DE)
(74) Vertreter: Kuhnen & Wacker Patent- und Rechtsanwaltsbüro PartG mbB

(57) **Zusammenfassung**

Die Erfindung betrifft ein Gurteinzugssystem für einen Prothesenschaft (12) aufweisend:
- einen Montageadapter (2) ausgebildet und eingerichtet zum temporären Anbringen an einem Stumpfmodell (6);
- eine Verriegelungstasse (3) ausgebildet und eingerichtet zum Einbinden in einen Prothesenschaft (12), die einen Gurtkanal (10) aufweist, wobei
- der Montageadapter (2) und die Verriegelungstasse (3) Mittel zum wenigstens rotatorisch formschlüssigen Festlegen des Montageadapters (2) und der Verriegelungstasse (3) zueinander um eine Rotationsachse (Ra) und/oder
- wenigstens der Montageadapter (2) Mittel zum wenigstens rotatorisch formschlüssigen Festlegen des Montageadapters (2) gegenüber dem Stumpfmodell (6) während der Herstellung des Prothesenschafts (12) aufweisen.

## Beschreibung

Die Erfindung betrifft ein Gurteinzugssystem für einen Prothesenschaft und eine Verriegelungstasse, einen Montageadapter und einen Schiebestopfen hierfür.

Bei einer herkömmlichen handwerklichen Prothesenherstellung zur Versorgung eines Gliedmaßenstumpfes wird zunächst im Gipsmodellierverfahren ein Prothesenschaft des Patienten angefertigt und nachgebildet. Dieser Prothesenschaft besitzt eine Außenkontur, die einerseits vom tatsächlichen Stumpf des Patienten abhängt und eine Materialzugabe aufweist, die einem Liner entspricht, der beim späteren Tragen der Prothese vom Patienten über den Stumpf gestülpt wird und der Patient mit Stumpf und Liner in den Prothesenschaft eintaucht. Um den Prothesenschaft in einfacher Art und Weise auf den Stumpf des Patienten ziehen zu können, besitzt ein solcher Liner an seinem freien Ende ein Zugelement, welches mit einem Gurt in Verbindung stehen kann, welcher durch einen Gurtkanal am Boden des Prothesenschafts nach außen geführt werden kann. Mit diesem Gurt kann dann der Patient von außen den Prothesenschaft auf den Liner ziehen.

Zur Herstellung und Einbettung eines solchen Gurtkanals ist es üblich, auf dem Stumpfmodell einen Montageadapter anzubringen, der eine Grundplatte aufweist und mit einer Mehrzahl von Schrauben am Stumpfende verdrehsicher befestigt wird. Die rotatorische Ausrichtung dieses Montageadapters ist beliebig, da der Montageadapter um eine Rotationsachse Ra rotationssymmetrisch ist.

Bevor die Laminierung des Prothesenschaftes über das Stumpfmodell erfolgt, wird auf dem Montageadapter eine Verriegelungstasse aufgesteckt, die schlussendlich nach der Prothesenschaftfertigung im Prothesenschaft verbleibt. Die Verriegelungstasse umfasst den Gurtkanal und ist rotatorisch, d. h. um die Rotationsachse Ra bezüglich des Montageadapters frei drehbar. Es ist Aufgabe eines erfahrenen Orthopädietechnikmeisters, die Positionierung des Montageadapters in lateraler und transversaler Richtung geeignet vorzunehmen, sowie dafür zu sorgen, dass die rotatorische Ausrichtung der Verriegelungstasse gegenüber dem Montageadapter derart korrekt erfolgt, dass eine schlussendlich resultierende Gurtführung für den Patienten bequem ist.

Bei einer derartigen Vorgehensweise besteht die Gefahr, dass die rotatorische Ausrichtung der in den Prothesenschaft einzuformenden Verriegelungstasse bezüglich des Montageadapters und somit auch bezüglich des Patientenstumpfes sich ungewollt verändert und somit ein Prothesenschaft gefertigt wird, der einen ungeeigneten und für den Anwender nachteiligen Gurtverlauf besitzt.

Die Positionierung des Montageadapters relativ zur Verriegelungstasse benötigt außerdem große Erfahrung, so dass derartige Tätigkeiten überwiegend durch hochqualifizierte, sehr erfahrene, orthopädietechnisch ausgebildete Mitarbeiter, z. B. langjährige Gesellen oder Meister, durchgeführt wird. Dies ist relativ teuer.

Im Falle eines unbeabsichtigten Verdrehens der Verriegelungstasse gegenüber dem Montageadapter muss die Ausrichtung durch derartig qualifizierte Personen erneut durchgeführt werden.

Des Weiteren sind gängige Gurteinzugssysteme der Gefahr unterworfen, beim Herstellungs-, d. h. beim Laminierungsprozess der Prothesenschäfte, ungewollt mit Harz vollzulaufen, so dass aufwendige Reinigungsarbeiten und umständliche Feinarbeiten am Ende der Herstellung erforderlich sind. Insbesondere muss teilweise mit Handfräsarbeiten am Grund des Prothesenschafts dafür gesorgt werden, dass ein Gurtende, welches am freien Ende des Liners befestigt ist, ausreichend Platz in der Verriegelungstasse findet, so dass ein leichtes und freigängiges Einziehen des Liners in den Prothesenschaft ermöglicht ist.

Ein derartiges System aus dem Stand der Technik ist beispielsweise aus der DE 10 2016 105 615 B3 bekannt.

Ein weiteres derartiges System, mit welchem die oben beschriebene gängige Prothesenschaftfertigung durchgeführt wird, zeigt die DE 10 2012 004 016 A1. Das dort gezeigte Gurteinzugssystem zeigt eine Verriegelungstasse, die einen Gurtkanal zur Verfügung stellt, wobei die Verriegelungstasse mit einem Prothesenaufnahmeanschluss ausgebildet ist. Die oben beschriebenen Nachteile treffen auch auf dieses System zu.

Des Weiteren sind Klicksysteme/Ratschensysteme für die Verbindung eines Liners für einen Patientenstumpf mit einem Prothesenschaft bekannt. Ein derartiges System zeigt beispielsweise die DE 20 2005 018 109 U1.

Aufgabe der Erfindung ist es daher, ein Gurteinzugssystem für einen Prothesenschaft anzugeben, welches einerseits vom Patienten leicht bedienbar und andererseits während der Herstellung des Prothesenschaftes eine erhebliche Vereinfachung und Verringerung der Fehleranfälligkeit im Herstellungsprozess gewährleistet.

Weiterhin ist es Aufgabe der Erfindung, ein Gurteinzugssystem als Montageset zur Verfügung zu stellen. Dies gelingt mit einer Verriegelungstasse mit den Merkmalen des Anspruchs 12, einen Montageadapter mit den Merkmalen des Anspruchs 13 und einem Schiebestopfen mit den Merkmalen des Anspruchs 14.

Alternativ gelingt dies auch durch das zur Verfügung stellen jeweils eines räumlichen Datenmodells einer Verriegelungstasse mit den Merkmalen des Anspruchs 15, einem räumlichen Datenmodell eines Montageadapters mit den Merkmalen des Anspruchs 16 und einem räumlichen Datenmodell eines Schiebestopfens mit den Merkmalen des Anspruchs 17.

Hinsichtlich der Aufgabenstellungen betreffend das Gurteinzugssystem wird die erfindungsgemäße Aufgabe durch ein Gurteinzugssystem für einen Prothesenschaft mit den Merkmalen des Anspruchs 1 gelöst. Vorteilhafte Ausführungsformen sind in den hiervon abhängigen Ansprüchen 2 bis 11 angegeben.

Ein Gurteinzugssystem für einen Prothesenschaft gemäß der Erfindung weist auf:
einen Montageadapter ausgebildet und eingerichtet zum temporären Anbringen an einem Stumpfmodell;
eine Verriegelungstasse ausgebildet und eingerichtet zum Einbinden in einen Prothesenschaft, die einen Gurtkanal aufweist, wobei
der Montageadapter und die Verriegelungstasse Mittel zum wenigstens rotatorisch formschlüssigen Festlegen des Montageadapters und der Verriegelungstasse zueinander um eine Rotationsachse Ra und/oder
wenigstens der Montageadapter Mittel zum wenigstens rotatorisch formschlüssigen Festlegen des Montageadapters gegenüber dem Stumpfmodell während der Herstellung des Prothesenschafts aufweisen.

Mit oben genanntem Gurteinzugssystem gelingt es, einerseits ein für Patienten besonders bequem anwendbares und mit wenig Kraftaufwand betätigbares Gurteinzugssystem zur Verfügung zu stellen, was den täglichen Gebrauch einer Prothese deutlich erleichtert.

Des Weiteren gelingt es, auf Seiten der Prothetik den Herstellungsprozess eines Prothesenschaftes deutlich zu vereinfachen und insbesondere die Fehleranfälligkeit des Herstellungsprozesses und somit den Ausschussgrad deutlich zu reduzieren.

In einer bevorzugten Ausführungsform der Erfindung ist das Gurteinzugssystem dadurch gekennzeichnet, dass der Montageadapter ausgebildet und eingerichtet ist, mittels einem einzigen Befestigungsmittel am Stumpfmodell befestigt zu werden und zur rotatorisch formschlüssigen Befestigung um die Rotationsachse Ra am Stumpfmodell wenigstens einen Vorsprung oder eine Ausnehmung aufweist, die dazu eingerichtet und ausgebildet ist, beim Befestigen des Montageadapters am Stumpfmodell einen um eine Achse A des Befestigungsmittels oder um die Rotationsachse Ra rotatorisch formschlüssigen Eingriff bezüglich des Stumpfmodells auszubilden.

Hierdurch wird der Montageaufwand des Montageadapters am Stumpfmodell deutlich vereinfacht. Im Falle erforderlicher Korrekturen ist dies ebenfalls im Vergleich zum Stand der Technik deutlich vereinfacht, da lediglich ein Befestigungsmittel erneut gelöst werden muss, um dann beispielsweise eine rotatorische Anpassung um die Rotationsachse Ra vornehmen und können und den Montageadapter erneut zu befestigen.

Des Weiteren ist durch die nicht rotationssymmetrische Ausbildung des Montageadapters bereits am Gipsmodell des Stumpfmodells ersichtlich, in welche Richtung der Gurtkanal am fertigen Prothesenschaft weisen wird, so dass bereits durch die rotatorische Ausrichtung des Montageadapters am Stumpfmodell hier in einfacher Art und Weise eine Positionierung stattfinden kann.

In einer weiteren bevorzugten Ausführung des Gurteinzugssystems weist der Montageadapter einen um die Rotationsachse Ra nicht symmetrischen Aufnahmedom zum Zusammenwirken mit einer korrespondierenden Domausnehmung in der Verriegelungstasse auf.

Durch diese Maßnahme gelingt es, eine eindeutige rotatorische Positionierung der Verriegelungstasse gegenüber dem Montageadapter sicherzustellen, ohne dass hierdurch Winkelfehler hinsichtlich einer Verdrehung um die Rotationsachse Ra passieren können.

Eine weitere bevorzugte Ausführungsform des erfindungsgemäßen Gurteinzugssystems ist dadurch gekennzeichnet, dass der Montageadapter eine freie Stirnfläche aufweist, die im montierten Zustand des Montageadapters und der Verriegelungstasse in eine Längsrichtung L des Gurtkanals weist.

Durch diese Maßnahme gelingt es, eine Anschlagfläche zur Verfügung zu stellen, die den Gurtkanal abdeckt und eine gute Zusammenwirkungsbasis für einen weiter unten näher beschriebenen Schiebestopfen bietet.

In einer besonderen Ausführungsform des erfindungsgemäßen Gurteinzugssystems ist ein solcher Schiebestopfen vorhanden, welcher zum temporären Verschließen des Gurtkanals zumindest während der Herstellung des Prothesenschaftes dient.

Durch einen solchen Schiebestopfen, der durch den Gurtkanal gesteckt werden kann, wird verhindert, dass im Herstellprozess flüssiges Laminierharz oder dergleichen andere Werkstoffe in den Gurtkanal gelangen können und somit eine ungewollte Verschmutzung oder Verengung des Gurtkanals bewirken können.

In einer anderen Ausführungsform weist der Schiebestopfen zusätzlich wenigstens einen Steckvorsprung auf, der mit einer entsprechenden Einsteckausnehmung des Montageadapters korrespondierend zusammensteckbar ist.

Hierdurch wird zusätzlich eine temporäre Verbindung und eine Positioniergenauigkeit des Montageadapters relativ zur Verriegelungstasse um die Rotationsachse Ra sichergestellt. Des Weiteren ist durch ein derartiges Zusammenstecken des Schiebestopfens und des Montageadapters eine gute Ausfüllung des Freiraums gewährleistet, der später, d. h. am fertigen Prothesenschaft, Platz bieten soll für den einzuziehenden Gurt am Ende des Liners.

Einer vorteilhaften Ausfüllung und Freihaltung des hierfür erforderlichen Bauraumes kann dadurch gewährleistet werden, dass die Domausnehmung der Verriegelungstasse korrespondierend zum Aufnahmedom ausgebildet ist und der Gurtkanal im Bereich der freien Stirnfläche in die Domausnehmung mündet.

Weiterhin ist es vorteilhaft, dass die Verriegelungstasse an ihrem Außenumfang wenigstens eine Entlüftungsrille oder einen Entlüftungskanal besitzt.

Hierdurch kann in einfacher Art und Weise bei der Herstellung des Prothesenschaftes Unterdruck zwischen den aufgelegten Gewebebahnen und einer diese umgebenden Folie eingebracht werden und das Laminat des Prothesenschaftes blasenfrei laminiert werden, da Vakuum gut in die Zwischenlagen eindringen kann. Die Entlüftungsrille bzw. der Entlüftungskanal dient hierzu zur einwandfreien vollflächigen Sicherstellung des Vakuums.

Weiterhin kann es vorteilhaft sein, dass die Entlüftungsrille und/oder der Entlüftungskanal im Bereich einer umlaufenden Fixierungsrinne für Materialbahnen oder tiefzuziehenden Thermoplast, die zur Herstellung des Prothesenschaftes erforderlich sind, angeordnet sind.

Hierdurch gelingt es besonders einfach, an der "Sammelstelle" der Materialbahnen oder Trennfolien zuverlässig Vakuum zwischen diese bringen zu können und somit eine blasenfreie Laminierung sicherzustellen.

Die Rotationsachse Ra ist bevorzugt derart ausgerichtet, dass sie in etwa (d. h. bis auf einige Grad genau) der Fügerichtung FR des Prothesenschaftes relativ zum Stumpf des Patienten entspricht. Da eine solche Achse im Bereich der Orthopädietechnik, insbesondere in dem vorliegenden Bereich der Prothesenschaftfertigung nicht genau definierbar ist, muss hier sinnvollerweise von einem "etwa"-Verlauf gesprochen werden. Eine gradgenaue Angabe ist alleine schon deshalb nicht möglich, weil patientenspezifisch unterschiedliche Weichteile vorhanden sind, die beim Anziehen des Stumpfes auch verformt werden können. Insofern muss hier auf eine fachmännische Bedeutung der Richtungsangaben abgehoben werden, da eine mathematisch und akademisch genaue Definition im Bereich der Orthopädietechnik in allgemeiner Art und Weise nicht sinnvoll möglich erscheint.

Im Folgenden wird die Erfindung anhand der Zeichnungen beispielhaft näher erläutert. Es zeigen:
Figur 1: eine Explosionsdarstellung eines erfindungsgemäßen Gurteinzugssystems in einer Seitenansicht mit einem Montageadapter, einer Verriegelungstasse und einem Schiebestopfen;
Figuren 2a-2d: den Montageadapter aus Figur 1 in einer weiteren Seitenansicht (Fig. 2a), in einer Schnittansicht (Fig. 2b), in einer ersten perspektivischen Ansicht (Fig. 2c) und in einer zweiten perspektivischen Ansicht (Fig. 2d);
Figuren 3a-3e: die Verriegelungstasse aus Figur 1 in einer weiteren Seitenansicht (Fig. 3a), in einer Schnittansicht (Fig. 3b) und eine Detailansicht aus Figur 2d (Fig. 3c), eine erste perspektivische Ansicht (Fig. 3d), eine zweite perspektivische Ansicht (Fig. 3e) und ein Detail auf Figur 3e (Fig. 3f);
Figur 4: eine perspektivische Ansicht auf den Schiebestopfen aus Figur 1;
Figuren 5a, 5b: das erfindungsgemäße Gurteinzugssystem gemäß Figur 1 in einem zusammengebauten Zustand in einer Seitenansicht (Fig. 5a) und in einer Schnittansicht entlang der Linie A-A (Fig. 5b);
Figur 6: ein Gipsmodell eines Patientenstumpfes (inkl. Linermaterialzugabe) mit aufgesetzten erfindungsgemäßen Gurteinzugssystem am freien Ende des Stumpfmodells;
Figur 7: eine Schnittdarstellung eines Prothesenschaftes, eines Liners und einer Funktionsdarstellung des Gurtes und dessen Führung durch den Gurtkanal des Gurteinzugssystems in einer Gesamtansicht;
Figur 8: eine Detailansicht H aus Figur 7.

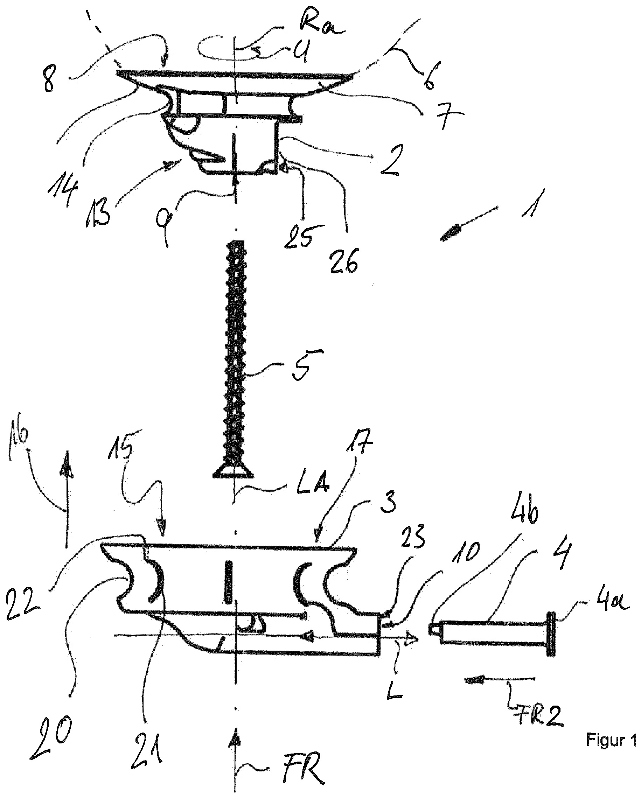

Ein erfindungsgemäßes Gurteinzugssystem 1, im Folgenden gelegentlich kurz als Einzugssystem 1 bezeichnet, zeigt Figur 1 in einer Explosionsdarstellung in einer Seitenansicht.

Das Gurteinzugssystem 1 weist wenigstens einen Montageadapter 2 und eine Verriegelungstasse 3 auf. Optional bzw. ergänzend zu den vorgenannten Mindestbestandteilen eines erfindungsgemäßen Gurteinzugssystems 1 kann ein Schiebestopfen 4 vorgesehen sein. Als Befestigungsmittel 5 zur Befestigung des Montageadapters 2 an ein Stumpfmodell 6 ist beispielsweise eine Schraube 5 vorgesehen. Die Schraube 5, d. h. das Befestigungsmittel 5, ist als Standard-/Normbauteil kein wesentlicher Bestandteil des Gurteinzugssystems 1, wird jedoch für dessen Anwendung benötigt.

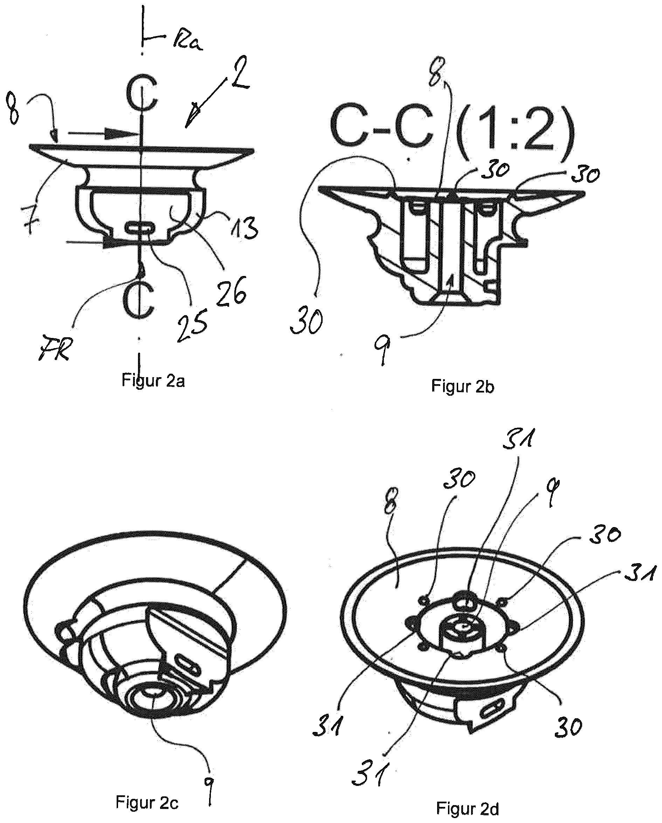

Erfindungsgemäß ist der Montageadapter 2 derart ausgebildet, dass er mittels lediglich einem einzigen Befestigungsmittel 5 am Stumpfmodell 6 rotatorisch um eine Rotationsachse Ra formschlüssig am Stumpfmodell 6 befestigbar ist. Das Stumpfmodell 6 besteht üblicherweise aus Gips oder einem anderen Modellierwerkstoff, in welchem das Befestigungsmittel 5 beispielsweise eingeschraubt werden kann. Mittel zum Sicherstellen des rotatorischen Formschlusses zwischen dem Montageadapter 2 und dem Stumpfmodell 6 sind in der Seitenansicht gemäß Figur 1 verdeckt und somit nicht dargestellt und werden weiter unten z. B. im Zusammenhang mit Figur 2d beschrieben werden.

Der Montageadapter 2 weist zur Kontaktierung bzw. zur Anlage am Stumpfmodell 6 einen Anlageteller 7 auf, welcher an seiner Oberseite 8, d. h. der Seite, die bestimmungsgemäß zum Stumpfmodell 6 hin weist, kalottenförmig ausgenommen ist.

Des Weiteren besitzt der Montageadapter 2 ein Durchgangsloch 9 zum Durchstecken des Befestigungsmittels 5. Das Durchgangsloch 9 fluchtet zweckmäßigerweise mit der Rotationsachse Ra bzw. einer Längsachse LA des Befestigungsmittels 5. Die Längsachse LA des Befestigungsmittels 5 ist in etwa parallel zu einer Fügerichtung FR der Einzelteile des Gurteinzugssystems 1 ausgerichtet. Die Fügerichtung FR bezieht sich dabei auf eine Fügerichtung der Verriegelungstasse 3 relativ zum Montageadapter 2. Eine zweite Fügerichtung FR2 steht im Wesentlichen senkrecht, insbesondere senkrecht auf der Fügerichtung FR und betrifft diejenige Fügerichtung, mit der der Schiebestopfen 4 in einen Gurtkanal 10 der Verriegelungstasse 3 einsteckbar ist.

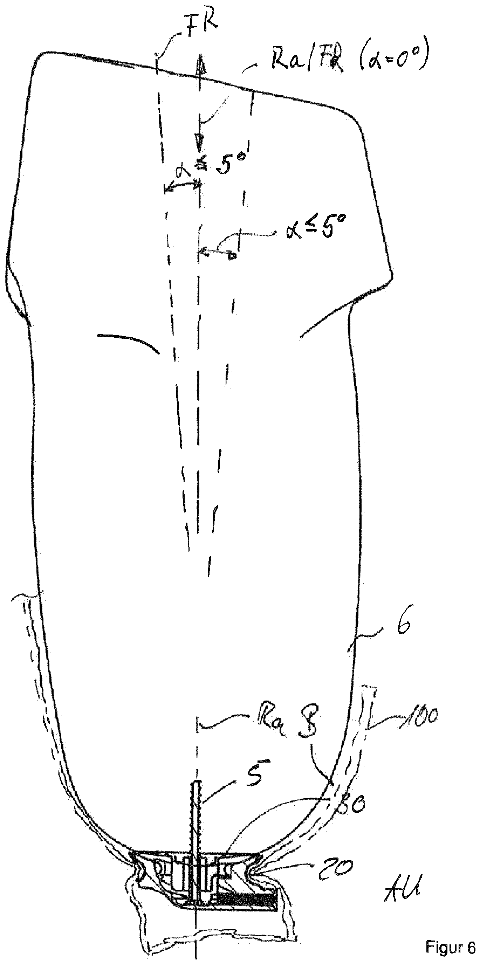

In der Ausführungsform gemäß Figur 1 ist eine Längsrichtung L des Gurtkanals 10 senkrecht zur Fügerichtung FR bzw. zur Längsachse LA ausgerichtet. Selbstverständlich ist es auch möglich, zwischen der Längsrichtung L und der Fügerichtung FR bzw. der Rotationsachse Ra einen Winkel ≠ 90° vorzusehen. Dies kann insbesondere dann sinnvoll sein, wenn ein seitlicher Gurtaustritt am herzustellenden Prothesenschaft 12 (nicht gezeigt in Figur 1; vergl. Figur 6 bis 8) abgewinkelt oder geneigt erfolgen soll.

Der Montageadapter 2 besitzt einen Aufnahmedom 13, welcher sich gegenüberliegend zur Oberseite 8 von dem Anlageteller 7 in Richtung der Längsachse LA bzw. in Richtung der Rotationsachse Ra vom Anlageteller 7 wegerstreckt. Der Aufnahmedom 13 ist dabei um die Längsachse LA bzw. um die Rotationsachse Ra nicht rotationssymmetrisch ausgebildet. Zwischen dem Aufnahmedom 13 und dem Anlageteller 7 ist vorzugsweise umlaufend in einer Umfangsrichtung U um die Rotationsachse Ra eine nach radial außen offene umlaufende Rinne 14 eingeformt. Eine derartige Rinne 14 ist jedoch entbehrlich und kann durch anderweitige geometrische Ausgestaltungen, z. B. durch einen entsprechenden Quaderblock, der keine umlaufende Rille aufweist, ersetzt werden.

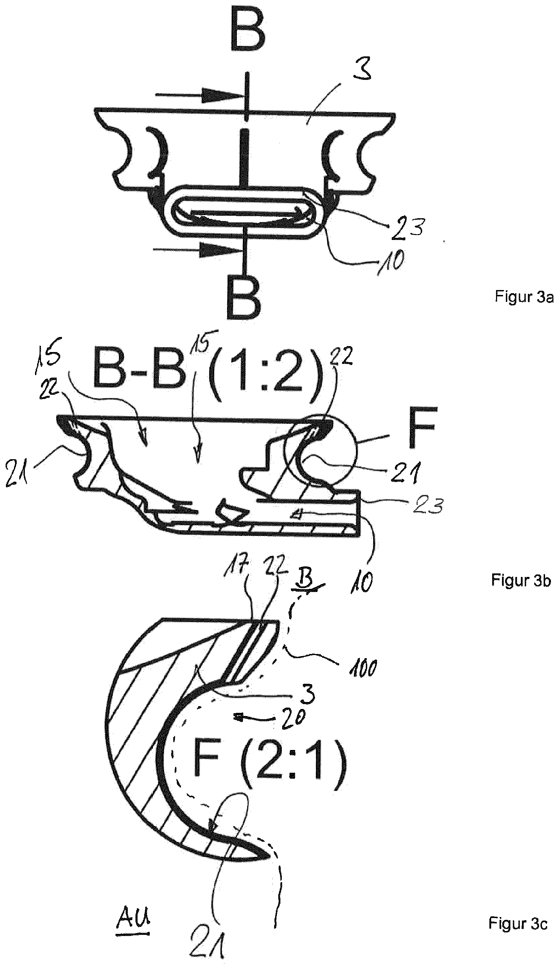

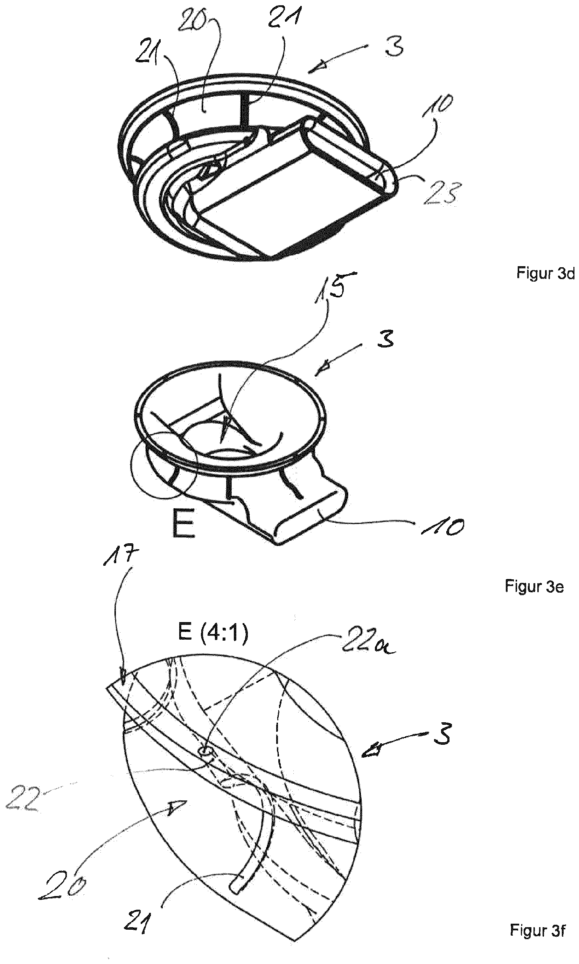

Die Verriegelungstasse 3 weist korrespondierend zum Aufnahmedom 13 eine Domausnehmung 15 (verdeckt in Figur 1; vergl. Figur 3b, 3e) auf. Die Domausnehmung 15 ist hinsichtlich ihrer dreidimensionalen Kontur derart ausgebildet, dass der Aufnahmedom 13 vollständig in der Domausnehmung 15 aufgenommen werden kann, wenn die Verriegelungstasse 3 in der Pfeilrichtung 16, die der Fügerichtung FR entspricht, auf den Montageadapter 2 zubewegt wird. Die Domausnehmung 15 ist zudem derart korrespondierend zum Aufnahmedom 13 ausgebildet, dass der Aufnahmedom 13 rotatorisch formschlüssig um die Rotationsachse Ra in der Domausnehmung 15 aufgenommen werden kann. Rotatorisch formschlüssig bedeutet dabei, dass eine wesentliche Verdrehung der Verriegelungstasse 3 gegenüber dem Montageadapter 2 nicht möglich ist. Jedoch kann ein zum problemlosen Fügen in Fügerichtung FR der Bauteile Montageadapter 2 und Verriegelungstasse 3 selbstverständlich ein geringes Spiel, welches beispielsweise ein Ausmaß einer groben Spielpassung haben kann, vorhanden sein. Wesentlich für einen rotatorischen Formschluss der Verriegelungstasse 3 relativ zum Montageadapter 2 ist, dass die Verriegelungstasse 3 gegenüber dem Montageadapter 2 nicht um die Rotationsachse Ra verdrehbar ist, wobei jedoch geringe, fachübliche Fügespiele hierbei unberücksichtigt bleiben sollen. Sinnvollerweise beträgt ein maximaler Verdrehwinkel in Umfangsrichtung U zwischen dem Montageadapter 2 und der Verriegelungstasse 3 aufgrund der entsprechenden Spielspalte zwischen dem Aufnahmedom 13 und der Domausnehmung 15 weniger als 2°, insbesondere weniger als 1°.

Korrespondierend zum Anlageteller 7 weist die Verriegelungstasse 3 an ihrer im bestimmungsgemäßen Gebrauch zum Montageadapter 2 weisenden Oberseite 17 einen zu einer Raumform des Anlagetellers 7 korrespondierende Raumform auf, so dass die Oberseite 17 wenigstens partiell flächig mit dem Anlageteller 7 in Kontakt kommt, wenn die Verriegelungstasse 3 auf dem Montageadapter 2 in Fügerichtung FR aufgesteckt ist.

Die Verriegelungstasse 3 weist außenseitig, d. h. in der Umfangsrichtung U umlaufend eine Fixierrinne 20 auf, welche dazu eingerichtet und ausgebildet ist, Bahnenmaterial und Lagenmaterial, welches zur Ausbildung eines Prothesenschafts während dessen Herstellung zunächst lose vorliegt, durch Abbindung in der Fixierrinne 20 festgelegt werden können. Die Fixierrinne 20 weist insbesondere am Rillengrund verlaufende Entlüftungsrillen 21 auf, welche es ermöglichen, dass ein Vakuum am Grund der Fixierrinne 20 von einer Außenumgebung in den Bereich zwischen Lagen/Gewebelagen oder einem tiefzuziehenden Thermoplast zur Ausbildung des Prothesenschafts vakuumgezogen werden sollen, hindurchlässt.

Wenigstens streckenweise kann wenigstens eine Entlüftungsrille 21 oder mehrere Entlüftungsrillen 21, die über den Umfang verteilt angeordnet sind, mittels eines Entlüftungskanals 22 eine Wandung, die zum Montageadapter 2 hinweist, durchdringen und im Bereich der Domausnehmung 15 bzw. der Oberseite 17 aus der Verriegelungstasse 3 austreten. Mit einer solchen Konstruktion gelingt es, das Vakuum, wenn es über die Entlüftungsrillen 21 entlang des Grundes der Fixierungen 20 geleitet werden kann, durch die geschlossenen Entlüftungskanäle 22 in den Innenbereich zwischen Stumpfmodell 6 und den vakuumzuziehenden Lagen zu verbringen.

Zum Verschließen des Gurtkanals 10 während der Herstellung des Prothesenschafts 12 ist es zweckmäßig, die Öffnung des Gurtkanals 10 zu verschließen, damit kein Gießharz oder dergleichen andere Verschmutzungen in den Gurtkanal 10 eindringen können. Hierzu dient der optionale Schiebestopfen 4, welcher entlang der Fügerichtung FR2 in den Gurtkanal 10 einsteckbar ist. Der Schiebestopfen 4 weist einen Anschlagteller 4a auf, welcher zum Anliegen an Endkanten 23 des Gurtkanals 10 ausgebildet sind und somit einen Verschluss des Gurtkanals 10 bewirkt.

An einem in FR2-Richtung vorderen Ende besitzt der Schiebestopfen 4 einen Steckvorsprung 4b, welcher in einer in Figur 1 gezeichneten Vertikalrichtung etwas nach oben versetzt, d. h. näher an einer Oberseite des Schiebestopfens 4 als an einer Unterseite des Schiebstopfens 4 angeordnet ist. Der Steckvorsprung 4b korrespondiert im eingesteckten Zustand des Schiebestopfens 4 in den Gurtkanal 10 und im aufgesteckten Zustand der Verriegelungstasse 3 auf den Montageadapter 2 mit einer Einsteckausnehmung 25 in einer dem Gurtkanal 10 zugewandten Flachseite 26 des Aufnahmedoms 13 des Montageadapters 2, so dass mit dem Einstecken des Schiebestopfens 4 bzw. dessen Steckvorsprungs 4b in die Einsteckausnehmung 25 eine Verriegelung der ineinandergefügten Bauteile Montageadapter 2 und Verriegelungstasse 3 entgegen der Fügerichtung FR bewirkt wird. Somit ist im eingesteckten Zustand des Schiebestopfens 4 die Verriegelungstasse 3 gegenüber dem Montageadapter 2 entgegen der Fügerichtung FR formschlüssig gegen Lösen gesichert.

Die Einsteckausnehmung 25 ist dabei in der Flachseite 26 als Sacklocköffnung korrespondierend zum Steckvorsprung 4 ausgebildet.

Im Folgenden wird der Montageadapter 2 anhand der Figuren 2a bis 2d insoweit ergänzend beschrieben, soweit dies nicht bereits im Zusammenhang mit Figur 1 geschehen ist.

In Figur 2a ist der Montageadapter 2 mit Blickrichtung auf die Flachseite 26 dargestellt, so dass die Einsteckausnehmung 25 deutlich sichtbar ist. Der Aufnahmedom 13 ragt dabei vom Anlageteller 7 derart weit entgegen der Fügerichtung FR vor, dass die Einsteckausnehmung 25 in einer Ansicht parallel zur Längsrichtung L des Gurtkanals 10 mit diesem fluchtet.

In Figur 2c ist eine Schnittdarstellung entlang der Linie C-C aus Figur 2a dargestellt. In dieser Schnittansicht ist das Durchgangsloch 9 (vgl. auch Figuren 2c und 2b) deutlich sichtbar. Das Durchgangsloch 9 ist etwa zentral mittig relativ zum Anlageteller 7 angeordnet und bietet einen Durchgang für das singuläre Befestigungsmittel 5.

Weiterhin sichtbar sind in der Schnittdarstellung gemäß Figur 2c eine Vielzahl von Vorsprüngen 30 (vgl. auch Figur 2b), welche gegenüber der Oberseite 8 des Anlagetellers 7, welche kalottenförmig ausgebildet ist, etwas überstehen. Wird nunmehr der Montageadapter 2 mittels des Befestigungsmittels 5 an das Stumpfmodell 6, welches beispielsweise aus Gips gefertigt ist, angeschraubt, so dringen die Vorsprünge 30 ein Stück in das Gipsmaterial ein und sorgen so für eine verdrehsichere Montage, d. h. eine formschlüssig verdrehsichere Montage des Montageadapters 2 am Stumpfmodell 6. Dies hat den besonderen Vorteil, dass im Vergleich zum Stand der Technik, an dem eine Mehrzahl von Befestigungsmitteln 5, z. B. Schrauben, vorzusehen sind, um den Montageteller 2 ordnungsgemäß am Stumpfmodell 6 zu befestigen, eingespart werden kann. Eine Kombination aus Vorsprüngen 30, d. h. irgendwie gearteten Eindringkörpern, die eingerichtet und ausgebildet sind, in eine Oberfläche des Stumpfmodells 6 eindringen zu können, in Kombination mit einem einzigen Befestigungsmittel 5 wurde erfindungsgemäß als ausreichend erkannt und erleichtert und vereinfacht die Montage des Montageadapters 2 am Stumpfmodell 6 erheblich.

Die Vorsprünge 30 sind dabei umlaufend angeordnet. Anstelle der Vorsprünge 30 oder zusätzlich zu den Vorsprüngen 30 können auch lokale Ausnehmungen 31 vorgesehen sein, welche ebenfalls zur verdrehsicheren, d. h. rotatorisch formschlüssigen Montage des Montageadapters 2 am Stumpfmodell 6 beitragen. Dies insbesondere dann, wenn beispielsweise Material des Stumpfmodells 6 an der Oberfläche noch deformierbar ist und ein dagegen gepresster Montageadapter 2 es dem Oberflächenmaterial des Stumpfmodells 6 bis zu dessen völligen Aushärtung ermöglicht, die Ausnehmung 31 zu befüllen und somit nach der Aushärtung eine um die Rotationsachse Ra formschlüssige Verbindung zwischen dem Montageadapter 2 und dem Stumpfmodell 6 zur Verfügung stellt.

In besonderer Art und Weise wurde erfindungsgemäß erkannt, dass der Montageadapter 2 als verlorenes Bauteil für die Herstellung von Prothesenschäften 12 besonders zweckmäßig als 3D-gedrucktes Bauteil ausgebildet sein kann. Selbstverständlich kann auch ein spritzgegossenes Bauteil, ein spritzgegossener Montageadapter oder ein anderweitig hergestellter Montageadapter 2 bevorzugt aber nicht ausschließlich aus Kunststoff eingesetzt werden. Insbesondere eignet sich ein 3D-Datenmodell des Montageadapters 2 dafür, beispielsweise virtuell an ein 3D-Datenstumpfmodell angeformt bzw. eingeformt zu werden. Dies wurde beispielsweise durch einen Körperscan des Patienten erhalten. Ein Einformen bzw. Anformen kann z. B. im Wege von virtuellen Verschneidungen zu erfolgen.

Sofern also ein Stumpfmodell 6 und ein Montageadapter 2 nicht mehr real als Gipsmodell und als realer Montageadapter aus Einzelteilen gebaut werden soll, sondern zunächst z. B. in einem CAD-System im Rechner virtuell zusammengestellt werden soll, kann der 3D-Datensatz eines auszudruckenden Montageadapters besonders hilfreich sein und mit einem Scan-Datensatz eines Patientenstumpfes vereinigt werden, so dass gegebenenfalls das Stumpfmodell 6 zusammen mit dem Montageadapter 2 als ein einziges integriertes Bauteil z. B. im Wege des 3D-Drucks ausgedruckt werden kann.

Die Verriegelungstasse 3 ist beispielhaft in den Figuren 3a - 3f näher erläutert.

Figur 3a zeigt eine Seitenansicht mit Blickrichtung in Fügerichtung FR auf die Verriegelungstasse 3 derart, dass in den dem Betrachter zugewandten Gurtkanal 10 geblickt werden kann.

Weiterhin sind die umlaufenden Endkanten 23 des Gurtkanals 10 sichtbar, gegen den der Schiebestopfen 4 mit seinem Anschlagteller 4a gesetzt werden kann.

Die übrigen Bestandteile wurden bereits im Zusammenhang mit Figur 1 beschrieben.

Figur 3d zeigt eine Schnittdarstellung entlang der Linie B-B durch die Verriegelungstasse 3. Hierdurch wird insbesondere der Gurtkanal 10 sichtbar, in dem der Schiebestopfen 4 einsteckbar ist.

Des Weiteren sind die Entlüftungskanäle 22 sichtbar, welche mit den Entlüftungsrillen 21 kommunizieren. Ein derartiger Kanalverlauf ist in Figur 3c als Detail F aus Figur 3b nochmals vergrößert dargestellt. Erkennbar ist, dass der Entlüftungskanal 22 randlich außen den Grundkörper der Verriegelungstasse 3 durchdringt und an der Oberseite 17 austritt. Somit entsteht über die Entlüftungsrillen 21, die entlang der Fixierrinne 20 bis zu den Entlüftungskanälen 22 laufen, ein Strömungskanal für Vakuum, welches somit von einer Außenumgebung AU in einen Bereich B zwischen einer Materiallage 100 des zu formenden Prothesenschafts 12 und dem Stumpfmodell 6 führt. Durch diesen Strömungskanal kann Vakuum in einfacher und prozesssicherer Art und Weise in den Bereich B geleitet werden.

Figur 3d zeigt eine perspektivische Unteransicht der Verriegelungstasse 3. Die angegebenen Bezugszeichen wurden bereits im Zusammenhang mit vorangegangenen Figuren allesamt beschrieben.

In Figur 3f ist in einer semitransparenten Darstellung nochmals der Verlauf einer offenen Entlüftungsrille 21, die entlang des Grundes der Fixierrinne 20 läuft und deren Übergang in einen umfänglich geschlossenen Entlüftungskanal 22 gezeigt, welcher mit einer Kanalöffnung 22a an der Oberseite 17 der Verriegelungstasse 3 austritt.

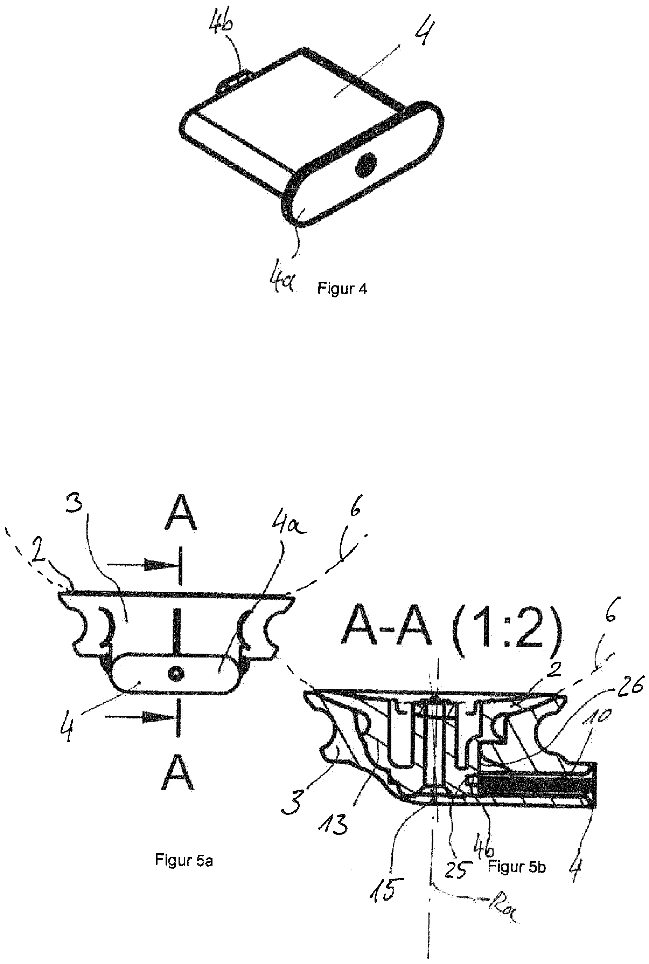

In Figur 4 ist nochmals eine perspektivische Ansicht auf den Schiebestopfen 4 dargestellt, dessen Steckvorsprung 4b an einer vorderen Stirnseite abgeht. Gegenüberliegend zum Steckvorsprung 4b ist der Anschlagteller 4a vorgesehen.

Figuren 5a und 5b zeigen ein erfindungsgemäßen Gurteinzugssystem 1 in einem zusammengebauten Zustand in einer Seitenansicht (Figur 5a) und in einer Schnittansicht entlang der Linie A-A (Figur 5b).

Die Seitenansicht gemäß Figur 5a entspricht hinsichtlich der Blickrichtung der Seitenansicht gemäß der Figur 3a. Der Gurtkanal 10 ist durch den Anschlagteller 4a des Schiebestopfens 4 verschlossen. Die Verriegelungstasse 3 ist über den Montageadapter 2 gesteckt, welcher am Stumpfmodell 6 angebracht ist. Zur Anbringung des Montageadapters 2 am Stumpfmodell 6 ist sinnvollerweise das Befestigungsmittel 5 vorgesehen, welches in den Figuren 5a, 5b nicht dargestellt ist. Der Aufnahmedom 13 des Montageadapters 2 sitzt in der Domausnehmung 15 der Verriegelungstasse 3 rotatorisch (bis auf evtl. vorhandene Spiele) formschlüssig. Der Gurtkanal 10 ist mittels des Schiebestopfens 4 verschlossen. Der Steckvorsprung 4b des Schiebestopfens 4 sitzt in der korrespondierenden Einsteckausnehmung 25 an der Flachseite 26 des Aufnahmedoms 13.

Figur 6 zeigt die Montagesituation eines erfindungsgemäßen Einzugssystems 1 gemäß Figur 5 in einer teilgeschnittenen Übersichtsansicht an einem Stumpfmodell 6. In dieser Ansicht ist das Befestigungsmittel 5 sichtbar, welches in das Stumpfmodell 6 eingeschraubt ist und im Zusammenspiel mit den Vorsprüngen 30 und/oder den Ausnehmungen 31 für eine rotatorisch formschlüssige Befestigung des Montageadapters 2 bezüglich des Stumpfmodells 6 sorgt.

Ausgehend von einem solchen präparierten Stumpfmodell 6, an den das Gurteinzugssystem 1 befestigt ist, können nunmehr Lagen, z. B. Materiallagen 100, welche zur Ausbildung bzw. zur Laminierung des herzustellenden Prothesenschaftes 12 dienen, in die Fixierrinne 20 eingeschnürt werden. Wie vorbeschrieben, kann über die Entlüftungsrillen 21 zusammen mit den Entlüftungskanälen 22 Vakuum von der Außenumgebung AU in den Bereich B zwischen den Lagen 100 und den Stumpfmodell 6 gelangen und somit ein Vakuum ziehen und ein Entlüften zur Gewährleistung eines engen Anliegens der Lagen 100 am Stumpfmodell 6 erfolgen.

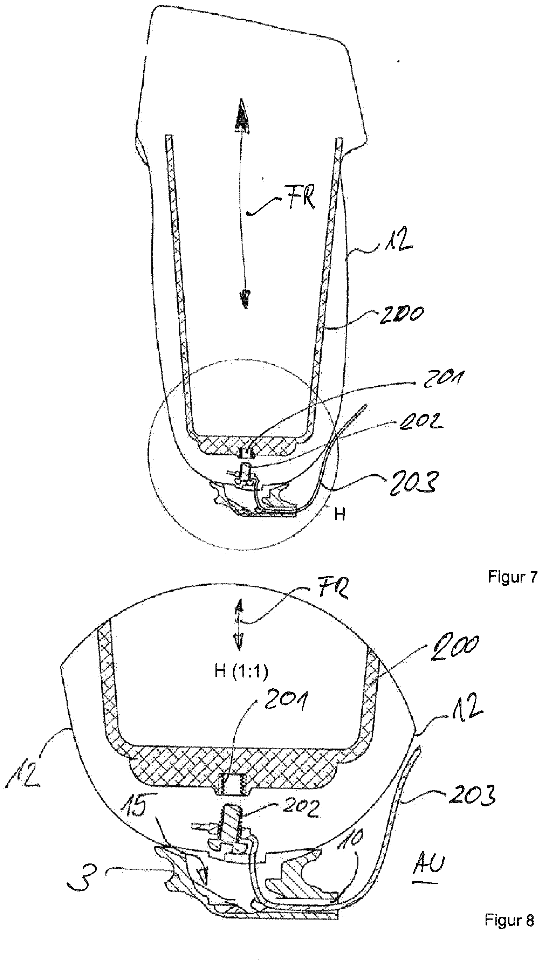

Figuren 7 und 8 zeigen ohne das Vorhandensein eventueller Lagen 100 die Funktionsweise des erfindungsgemäßen Gurteinzugssystems 1 unter der Annahme, dass die Verriegelungstasse 3 am Prothesenschaft 12 integriert ist. Ein Liner 200, der über einen nicht gezeigten Patientengliedmaßenstumpf gestülpt wird, weist an seinem unteren Ende eine Anschraubstelle 201 für eine Zentralschraube 202 auf. Eine solche Zentralschraube 202 ist beispielsweise und bevorzugt als Flachkopfschraube ausgebildet, die mit dem Innenraum der Domausnehmung 15 der Verriegelungstasse 3 korrespondiert und dort genug Platz findet. Die Zentralschraube 202 befestigt einen Gurt 203 am Liner 200. Der Gurt 203 ist von der Befestigungsstelle am Liner 200 durch den Gurtkanal 10 der Verriegelungstasse 3 geführt und kann (wenn die Zentralschraube 202 an der Anschraubstelle 201 befestigt ist) bewirken, dass durch ziehen am Gurt 203 der Prothesenschaft 12 relativ zum Liner 200 in der Fügerichtung FR bewegt wird und somit "über den Liner 200 gezogen wird". Diese Einzugshilfe, insbesondere das erfindungsgemäße Einzugssystem 1 zeichnet sich dabei dadurch aus, dass durch die angepasste Innengestaltung der Domausnehmung 15 ein geschmeidiges Einziehen der Zentralschraube 202 erfolgt und eine geschmeidige Gurtführung aus der Domausnehmung 15 durch den Gurtkanal 10 in die Außenumgebung AU gewährleistet ist.

In den Figuren 7 und 8 ist ein fertiger Prothesenschaft 12, in den das erfindungsgemäße Gurteinzugssystem 1 integriert ist, gezeigt. Am fertigen Prothesenschaft 12 verbleibt vom erfindungsgemäßen Gurteinzugssystem 1 lediglich die Verriegelungstasse 3. Der für die Herstellung des Prothesenschafts 12 erforderliche Montageadapter 2 und der für die Herstellung des Prothesenschafts 12 vorteilhaft zu verwendende Schiebestopfen 4 verbleiben als wiederverwendbare Bauteile in der Werkstatt des Orthopädietechnikers und können bei der Herstellung eines anderen Prothesenschafts 12 für einen anderen Patienten erneut verwendet werden. Lediglich die Verriegelungstasse 3 verbleibt "einlaminiert" am Prothesenschaft 12 und dient nunmehr als Gurteinzugsvorrichtung 1.

### Bezugszeichenliste

- 1: Gurteinzugssystem/Einzugssytem
- 2: Montageadapter
- 3: Verriegelungstasse
- 4: Schiebestopfen
- 4a: Anschlagteller
- 4b: Steckvorsprung
- 5: Befestigungsmittel
- 6: Stumpfmodell
- 7: Anlageteller
- 8: Oberseite
- 9: Durchgangsloch
- 10: Gurtkanal
- 12: Prothesenschaft
- 13: Aufnahmedom
- 14: Rinne
- 15: Domausnehmung
- 16: Pfeilrichtung
- 17: Oberseite
- 20: Fixierrinne
- 21: Entlüftungsrille
- 22: Entlüftungskanal
- 22a: Kanalöffnung
- 23: Endkante
- 25: Einsteckausnehmung
- 26: Flachseite
- 30: Vorsprung
- 31: Ausnehmung
- 100: Materiallage
- 200: Liner
- 201: Anschraubstelle
- 202: Zentralschraube
- 203: Gurt

- A: Achse
- AU: Außenumgebung
- B: Bereich
- L: Längsrichtung
- LA: Längsachse
- FR: Fügerichtung
- FR2: zweite Fügerichtung
- Ra: Rotationsachse
- U: Umfangsrichtung

- α: Winkel

## Patentansprüche

1. Gurteinzugssystem für einen Prothesenschaft (12) aufweisend:
- einen Montageadapter (2) ausgebildet und eingerichtet zum temporären Anbringen an einem Stumpfmodell (6);
- eine Verriegelungstasse (3) ausgebildet und eingerichtet zum Einbinden in einen Prothesenschaft (12), die einen Gurtkanal (10) aufweist, wobei
- der Montageadapter (2) und die Verriegelungstasse (3) Mittel zum wenigstens rotatorisch formschlüssigen Fest-legen des Montageadapters (2) und der Verriegelungstasse (3) zueinander um eine Rotationsachse (Ra) und/oder
- wenigstens der Montageadapter (2) Mittel zum wenigstens rotatorisch formschlüssigen Festlegen des Montageadapters (2) gegenüber dem Stumpfmodell (6) während der Herstellung des Prothesenschafts (12) aufweisen.

2. Gurteinzugssystem nach Anspruch 1, **dadurch gekennzeichnet, dass** der Montageadapter (2) ausgebildet und eingerichtet ist, mittels einem einzigen Befestigungsmittel (5) am Stumpfmodell (6) befestigt zu werden und zur rotatorisch formschlüssigen Befestigung um die Rotationsachse (Ra) am Stumpfmodell (6) wenigstens einen Vorsprung (30) oder eine Ausnehmung (31) aufweist, die da-zu eingerichtet und ausgebildet ist, beim Befestigen des Montageadapters (2) am Stumpfmodell (6) einen um eine Achse (A) des Befestigungsmittels (5) oder um die Rotationachse (Ra) rotatorisch formschlüssigen Eingriff bezüglich des Stumpfmodells (6) auszubilden.

3. Gurteinzugssystem nach Anspruch 2, **dadurch gekennzeichnet, dass** der Montageadapter (2) einen um die Rotationsachse (Ra) nicht symmetrischen Aufnahmedom (13) zum Zusammenwirken mit einer korrespondierenden Domausnehmung (15) in der Verriegelungstasse (3) aufweist.

4. Gurteinzugssystem nach einem der vorangehenden Ansprüche, **dadurch gekennzeichnet, dass** der Montageadapter (2) eine freie Stirnfläche aufweist, die im montierten Zustand des Montageadapters (2) und der Verriegelungstasse (3) in eine Längsrichtung (L) des Gurtkanals (10) weist.

5. Gurteinzugssystem nach einem der vorangehenden Ansprüche, **dadurch gekennzeichnet, dass** das Einzugssystem (1) einen Schiebestopfen (4) zum temporären Verschließen des Gurtkanals (10) zumindest während der Herstellung des Prothesenschaftes (12) umfasst.

6. Gurteinzugssystem nach einem der vorangehenden Ansprüche, **dadurch gekennzeichnet, dass** der Schiebestopfen (4) wenigstens einen Steckvorsprung (4b) aufweist, der mit einer Einsteckausnehmung (25) des Montageadapters (2) korrespondierend zusammensteckbar ist.

7. Gurteinzugssystem nach einem der vorangehenden Ansprüche, **dadurch gekennzeichnet, dass** die Domausnehmung (15) der Verriegelungstasse (3) korrespondierend zum Aufnahmedom (13) ausgebildet ist und der Gurtkanal (10) im Bereich der freien Stirnfläche in die Domausnehmung (15) mündet.

8. Gurteinzugssystem nach einem der vorangehenden Ansprüche, **dadurch gekennzeichnet, dass** die Verriegelungstasse (3) an ihrem Außenumfang wenigstens eine Entlüftungsrille (21) und/oder einen Entlüftungskanal (22) besitzt.

9. Gurteinzugssystem nach einem der vorangehenden Ansprüche, **dadurch gekennzeichnet, dass** die Entlüftungsrille (21) und/oder der Entlüftungskanal (22) im Bereich einer umlaufenden Fixierrinne (20) für Materialbahnen oder eines tiefzuziehenden Thermoplast, die zur Herstellung des Prothesenschafts (12) erforderlich sind, angeordnet sind.

10. Gurteinzugssystem nach einem der vorangehenden An-sprüche, **dadurch gekennzeichnet, dass** die Rotationsachse (Ra) parallel oder im Wesentlichen parallel zu einer Fügerichtung (FR) des Prothesenschafts (12) relativ zum Stumpfmodell (6) oder zum Patientenstumpf verläuft oder die Rotationsachse (Ra) einer Längsachse (LA) des einzigen Befestigungsmittels (5) entspricht.

11. Gurteinzugssystem nach einem der vorangehenden An-sprüche, **dadurch gekennzeichnet, dass** ein Winkel α zwischen der Rotationsachse (Ra) und der Fügerichtung (FR) oder zwischen der Rotationsachse (Ra) und der Längsachse (LA) in Medial-/Lateralrichtung und/oder in Dorsal-/Ventralrichtung in einem Bereich zwischen 0° und ±5°, bevorzugt zwischen 0° und ±3° liegt.

12. Verriegelungstasse zur Ausbildung eines Einzugssystems (1) nach einem der Ansprüche 1 bis 11.

13. Montageadapter zur Ausbildung eines Einzugssystems (1) nach einem der Ansprüche 1 bis 11.

14. Schiebestopfen zur Ausbildung eines Einzugssystems (1) nach einem der Ansprüche 1 bis 11.

15. Räumliches Datenmodell einer Verriegelungstasse (3) zur Ausbildung eines Einzugssystems (1) nach einem der Ansprüche 1 bis 11, insbesondere geeignet zur Herstellung der Verriegelungstasse (3) mittels eines 3D-Druckverfahrens.

16. Räumliches Datenmodell eines Montageadapters (2) zur Ausbildung eines Einzugssystems (1) nach einem der Ansprüche 1 bis 11, insbesondere geeignet zur Herstellung des Montageadapters (2) mittels eines 3D-Druckverfahrens.

17. Räumliches Datenmodell eines Schiebestopfens (4) zur Ausbildung eines Einzugssystems (1) nach einem der Ansprüche 1 bis 11, insbesondere geeignet zur Herstellung des Schiebestopfens (4) mittels eines 3D-Druckverfahrens.
